# EUROPÄISCHE PATENTSCHRIFT

(11) **EP 0 425 472 B1**
(45) Veröffentlichungstag und Bekanntmachung des Hinweises auf die Patenterteilung: **10.08.1994**
(21) Anmeldenummer: 90890283.6
(22) Anmeldetag: 17.10.1990
(51) Int. Cl.: A61B 17/58

(54) **Knochennagel und Werkzeug für denselben**
Bone nail and tool therefor
Broche pour os et outil correspondant

(30) Priorität: 25.10.1989 AT 2463/89
(43) Veröffentlichungstag der Anmeldung: 02.05.1991
(73) Patentinhaber: Ender, Hans Georg, Dr., A-1180 Wien (AT)
(72) Erfinder: Ender, Hans Georg, Dr., A-1180 Wien (AT)
(74) Vertreter: Brauneiss, Leo, Dipl.Ing.

(56) Entgegenhaltungen:
- EP-A- 0 069 048
- EP-A- 0 094 489
- EP-A- 0 194 014
- WO-A-87/02572
- DE-A- 1 933 440
- DE-A- 3 526 742

## Beschreibung

Die Erfindung betrifft einen zumindest in seinem proximalen Endbereich gekrümmten Knochennagel aus elastischem Material mit rundem oder ovalem Querschnitt zur Fixierung von Brüchen im proximalen Oberschenkelbereich, welcher Nagel über ein proximal des Kniegelenkbereiches im Knochen angeordnetes Einschlagloch in den Markraum des Knochens eingeführt werden kann und infolge seiner Elastizität mit dem Krümmungsscheitel an der dem Einschlagloch gegenüberliegenden Wand des Markraumes unter Spannung anliegen kann, und dessen distaler Endbereich denselben Querschnitt aufweist wie der gesamte Nagel und zur Ausbildung als Kupplungsteil für die Herstellung einer allseits drehfesten Verbindung mit einem Werkzeug an zumindest einer Stelle des Nagelumfanges in Abstand vom distalen Nagelende mit einer Abflachung versehen ist. Ferner betrifft die Erfindung eine Kombination eines Werkzeuges und eines solchen Knochennagels.

Es ist bereits bekannt, per- und subtrochantere Frakturen dadurch zu reponieren und zu fixieren, daß der Markraum des Knochens durch ein Einschlagloch eröffnet wird und in dieses Einschlagloch zumindest ein Knochennagel, zweckmäßig mehrere Knochennägel, eingeführt wird bzw. werden, wobei die Knochennägel aus einem elastischen Material bestehen und wenigstens in ihrem proximalen Bereich gekrümmt sind. Werden diese Knochennägel in den Markraum eingeführt, so liegen sie infolge ihrer Elastizität mit dem Krümmungsscheitel an der dem Einschlagloch gegenüberliegenden Wand des Markraumes unter Spannung an, wobei dann, wenn die proximale Spitze jedes Nagels in den Bereich der Bruchstelle kommt, der Nagel über diese Bruchstelle in den Gelenkskopf des Knochens eintritt und die Bruchstelle fixiert. Durch Drehen der einzelnen Nägel können die Knochenteile an der Bruchstelle reponiert werden, so daß sie ihre richtige Lage relativ zueinander einnehmen. Hiezu ist das distale Ende jedes Nagels mit einem Kupplungsteil versehen, der eine allseits drehfeste Verbindung mit einem Werkzeug zum Einschlagen des Nagels ermöglicht.

Es ist bereits bekannt, diesen Kupplungsteil als hakenförmige Umbiegung auszubilden. Nachteilig ist bei dieser bekannten Ausführungsform, daß die hakenförmige Umbiegungen der Nägel weit abstehen, die Sehnen und Muskel irritieren und das Abbiegen des Beines im Bereich des Kniegelenks behindern. Außerdem sind bei Anwendung mehrerer Knochennägel, wie dies die Regel ist, diese hakenförmigen Umbiegungen einander im Wege. Schließlich drücken die hakenförmigen Umbiegungen infolge der unter Spannung im Knochen befindlichen elastischen Nägel mit beträchtlicher Kraft auf eine relativ kleine Stelle an der Außenseite des Knochens, so daß an dieser Stelle die spezifische Flächenpressung groß und daher insbesondere bei älteren Personen mit porotischen Knochen die Gefahr eines Einbrechens des Knochens besteht.

Man hat auch bereits vorgeschlagen, den Kupplungsteil als plättchenförmige Abflachung des distalen Nagelendes auszubilden. Bei dieser Ausbildung benötigt der Kupplungsteil weniger Platz, die einzelnen plättchenförmigen Abflachungen können sich dachziegelartig übereinanderlegen und behindern einander nicht, und die spezifische Flächenpressung wird durch die Vergrößerung der Fläche verkleinert, so daß die Gefahr eines Einbrechens des Knochens im Bereich der an der Knochenaußenseite anliegenden flachen Plättchen verkleinert, jedoch nicht vollständig beseitigt wird. Außerdem irritieren auch diese Plättchen, die aus dem Einschlagloch herausragen, die über diesem Einschlagloch laufenden Sehnen und Muskel.

Weiters ist ein zumindest in seinem proximalen Endbereich gekrümmter Knochennagel aus elastischem Material bekannt, dessen distaler Endbereich denselben Querschnitt aufweist wie der gesamte Nagel und an zumindest einer Stelle des Nagelumfanges in Abstand vom distalen Nagelende mit einer Abflachung versehen ist, wodurch dieser Endbereich als Kupplungsteil ausgebildet ist, der eine allseits drehfeste Verbindung mit einem zum Einschlagen des Nagels dienenden Werkzeug ermöglicht. Bei dieser Ausbildung weist der distale Endbereich somit keine vergrößerten Abmessungen auf und ermöglicht trotzdem die Herstellung einer drehfesten Verbindung mit dem erwähnten Werkzeug zum Einschlagen des Nagels. Die distalen Nagelenden können daher im Führungskanal eines bekannten, im Einschlagloch des Knochens fixierten Einsatzstückes untergebracht werden, dessen Führungskanal infolge der nicht verbreiterten distalen Endbereiche der Nägel kleine Abmessungen aufweisen kann. Ein solches Einsatzstück verhindert beim Einschlagen der Nägel, daß das Einschlagloch im Knochen aussplittert und dadurch in nachteiliger Weise vergrößert wird, und ermöglicht es, die distalen Enden der Nägel im Führungskanal unterzubringen, so daß sie nicht mehr aus dem Knochen herausragen. Dadurch wird eine Irritation der Muskel und Sehnen vermieden und es wird ein Einbrechen des Knochens im Bereich des Einschlagloches verhindert, da ja das als Kupplungsteil ausgebildete distale Ende der Nägel nicht mehr unter Spannung an der Knochenaußenseite anliegt, sondern die Kräfte vom Einsatzstück aufgenommen und von diesem gleichmäßig entlang des gesamten Umfanges des Einschlagloches auf den Knochen übertragen werden.

Bei aus der EP-A - 0 094 489 bekannten, mit wenigstens einer Abflachung am distalen Nagelende versehenen Knochennägeln sind die Stirnflächen, über welche die Abflachung in den Umfang des Knochennagels übergeht, senkrecht zur Abflachung verlaufend ausgebildet. Es müssen daher bei dieser bekannten Ausbildung entweder zusätzliche Öffnungen am distalen Nagelende für die Kupplung mit einem enstprechend geformten Werkzeug vorgesehen sein oder es müssen jene Teile des Werkzeuges, welche mit der Abflachung zusammenwirken, durch eine Überschubhülse fixiert werden, um ein Abgleiten von der Abflachung zu verhindern. Diese Überschubhülse vergrößert den Umfang des Werkzeuges in jenem Bereich, wo die Kupplung desselben mit dem als Kupplungsteil ausgebildeten distalen Nagelende erfolgt, beträchtlich. Wenn nun die distalen Nagelenden im Führungskanal des erwähnten Einsatzstückes aufgenommen sind, der kleine Abmessungen aufweist, so ist die Kupplung des Werkzeuges mit dem distalen Nagelende aus Platzgründen schwierig und zuweilen nicht möglich. Dies stellt vor allem beim Zurückschlagen der Nägel zwecks Entfernung derselben aus dem Markraum einen wesentlichen Nachteil dar. Während nämlich beim Einschlagen der Nägel in den Markraum in der letzten Phase des Einschlagvorganges, in welcher die distalen Nägelenden in den Führungskanal eintreten, ein Werkzeug verwendet werden kann, das lediglich mit dem stirnseitigen Nagelende zusammenwirkt, bei dem also eine Kupplung mittels der Abflachung nicht mehr erforderlich ist, muß zum Zurückschlagen der Knochennägel zwecks Entfernung derselben aus dem Markraum unbedingt eine Verbindung zwischen dem Nagel und dem zum Zurückschlagen benötigten Werkzeug über die Abflachung erfolgen.

Aus der DE-A-1 933 440 ist eine Vibrationseinrichtung für das Einführen und Herausziehen von Knochennägeln bekanntgeworden, die einen mit dem Knochennagel zu verbindenden Schaft aufweist, der mit einem hakenförmigen Aufsatz zum Entfernen langer Knochennägel verbunden werden kann. Die Art der Verbindung dieses hakenförmigen Aufsatzes mit den Knochennägeln ist nicht offenbart.Bei Kenntnis des Standes der Technik liegt es für den Fachmann nahe, diesen hakenförmigen Aufsatz zum Eingreifen in das distale Loch üblicher Knochennägel zu verwenden.

Die vorliegende Erfindung hat sich zur Aufgabe gestellt, einen Knochennagel der eingangs beschriebenen Art derart zu verbessern, daß eine Verbindung des distalen Nagelendbereiches mit einem Werkzeug hergestellt werden kann, dessen Abmessungen an der Verbindungsstelle klein gehalten werden können, und mit dem auch ein Herausziehen der Nägel aus dem Markraum vorgenommen werden kann. Zur Lösung dieser Aufgabe schlägt die Erfindung vor, daß zumindest die dem distalen Nagelende unmittelbar benachbarte Stirnfläche, über welche die Abflachung in den Umfang des Knochennagels übergeht, vom Umfang des Knochennagels ausgehend zur Abflachung in Richtung zum distalen Nagelende geneigt ausgebildet ist, somit mit dieser Abflachung einen von 90° abweichenden Winkel einschließt.
Durch die in Richtung zum distalen Nagelende geneigte Ausbildung dieser Stirnfläche wird verhindert, daß der mit der Abflachung zusammenwirkende Teil des Werkzeuges beim Einschlagen, aber vor allem beim Herausziehen des Nagels mittels dieses Werkzeuges von der Abflachung abgleitet und sich somit vom Knochennagel löst, auch wenn keine zusätzlichen Maßnahmen für die Fixierung dieses Teiles des Werkzeuges vorgesehen sind. Die erfindungsgemäße Ausbildung ermöglicht somit eine Kupplung zwischen den im Führungskanal eines Einsatzstückes befindlichen distalen Nagelenden und einem geeigneten Werkzeug zwecks Entfernen der Nägel aus dem Markraum, auch dann, wenn mehrere Nägel im Markraum angeordnet sind und die Abmessungen des Führungskanales eines Einsatzstückes klein sind, also wenig Platz für das Einführen des Werkzeuges in den Führungskanal des Einsatzstückes zur Verfügung steht.

Es ist möglich, lediglich die dem distalen Nagelende unmittelbar benachbarte Stirnfläche geneigt auszubilden, die gegenüberliegende Stirnfläche jedoch senkrecht zur Abflachung verlaufen zu lassen. Eine besonders vorteilhafte Ausführungsform ergibt sich jedoch dann, wenn beide gegenüberliegende Stirnflächen, über welche die Abflachung in den Umfang des Knochennagels übergeht, zueinander parallel und zur Abflachung geneigt ausgebildet sind und dann eine Arretierung für den mit der Abflachung zusammenwirkenden Teil des Werkzeuges bilden.

Der Winkel, den die geneigte Stirnfläche mit der Abflachung einschließt, beträgt zweckmäßig zwischen 30 und 60°, vorzugsweise etwa 45°.

Bei der erfindungsgemäßen Ausbildung des Knochennagels genügt zur Herstellung einer Verbindung mit einem Werkzeug das Zusammenwirken einer einzigen Abflachung mit dem entsprechenden Werkzeug. Fallweise ist es jedoch zweckmäßig, wenn die Möglichkeit besteht, das Werkzeug an verschiedenen Stellen mit dem distalen Endbereich des Nagels verbinden zu können, um die Lage des Werkzeuges den jeweiligen Erfordernissen anzupassen. Zu diesem Zweck können zwei oder mehrere in Umfangsrichtung des Nagels und/oder in Nagellängsrichtung versetzt angeordnete Abflachungen vorgesehen sein.

Wenn lediglich eine einzige mit einem Werkzeug zusammenwirkende Abflachung in der Umfangsfläche des distalen Endbereiches vorgesehen ist, so ist es ohne zusätzliche Maßnahmen schwierig, über diese einzige Abflachung eine sichere drehfeste Verbindung mit dem Werkzeug herzustellen, wie sie zum Reponieren der Bruchstelle erforderlich ist. Um diesen Nachteil zu beseitigen, ohne jedoch die Abmessungen des distalen Nagelendes zu vergrößern, ist gemäß einem weiteren Merkmal der Erfindung das distale Nagelende mit wenigstens einer von der Umfangsfläche des Nagels einspringenden, gegen das Nagelende offenen Ausnehmung versehen. Diese Ausnehmung, über welche, da sie gegen das Nagelende offen ist, ein entsprechend ausgebildetes Werkzeug auch dann geschoben werden kann, wenn der vorhandene Platz gering ist, ermöglicht eine sichere drehfeste Verbindung dieses Werkzeuges mit dem distalen Nagelende.

Gemäß einer bevorzugten Ausführungsform der Erfindung können an zwei diametral gegenüberliegenden Umfangsflächen des Knochennagels Ausnehmungen vorgesehen sein, von welchen ein gegen das distale Nagelende gerichteter Vorsprung begrenzt ist.

Die Begrenzungsfläche der Ausnehmung bzw. Ausnehmungen kann hiebei zumindest teilweise parallel oder senkrecht zur Abflachung verlaufen.

Gemäß einer Ausführungsform der Erfindung geht die Begrenzungsfläche der Ausnehmung über eine Abrundung in die Umfangsfläche des Nagels über, wodurch Spannungsspitzen vermieden werden. Es kann aber auch die Begrenzungsfläche der Ausnehmung über eine geneigte Fläche in die Umfangsfläche des Nagels übergehen, wobei deren Neigung vorzugsweise gleich ist jener der geneigten Stirnfläche, über welche die Abflachung in die Umfangsfläche des Nagels übergeht. Dadurch wird eine sichere Verbindung des Nagels mit dem Werkzeug erzielt.

Bei Belastung des gebrochenen Beines, in dessen Markraum sich die Knochennägel befinden, sinken die Fragmente zusammen. Um zu vermeiden, daß dadurch die Spitze des proximalen Nagelendes den Gelenkskopf perforiert, ist es zweckmäßig, wenn die im Markraum eingesetzten Knochennägel bei Belastung des gebrochenen Beines etwas nach distal verschoben werden. Hiebei gleitet die der Abflachung diametral gegenüberliegende Fläche des distalen Nagelendes an der Wand des Führungskanales des Einsatzstückes. Um zu verhindern, daß sich bei diesem Gleitvorgang das distale Nagelende im Führungskanal verklemmt und dann das Gleiten behindert wird, ist gemäß einem weiteren Merkmal der Erfindung das distale Nagelende im Querschnitt in einer in Nagellängsrichtung verlaufenden Normalebene auf die Abflachung von einer geneigt zur Abflachung verlaufenden Kurve begrenzt, wobei die Neigung der Kurve vorzugsweise entgegengesetzt der Neigung der dem distalen Nagelende benachbarten Stirnfläche verläuft. Diese Kurve erleichtert das Gleiten des distalen Nagelendes entlang der Fläche des Führungskanales.

Die Kombination eines Werkzeuges und eines erfindungsgemäßen Knochennagels ist im wesentlichen dadurch gekennzeichnet, daß der Werkzeugschaft mit einem hakenartigen, der Form wenigstens einer Stirnfläche, über welche die Abflachung in die Umfangsfläche des Nagels übergeht, angepaßten Ansatz aufweist. Dieser hakenartige Ansatz hintergreift beim Herausziehen des Knochennagels aus dem Markkanal die geneigte Stirnfläche, wodurch ein Abgleiten des Werkzeuges verhindert wird. Um mit dem erfindungsgemäßen Werkzeug den Knochennagel auch sicher in den Markkanal einführen zu können, geht gemäß einem weiteren Merkmal der Erfindung der hakenartige Ansatz über eine der Form des distalen Nagelendes angepaßte gekrümmte Fläche in den Werkzeugschaft über. Diese Ausbildung ermöglicht beim Einschlagen der Nägel eine Kraftübertragung in Richtung der Nagelachse, auch wenn das Werkzeug schräg angesetzt werden muß.

Um ein Herausgleiten des hakenförmigen Ansatzes aus der von den Stirnflächen begrenzten Abflachung mit Sicherheit zu verhindern, ist es von Vorteil, wenn der Werkzeugschaft an der dem hakenförmigen Ansatz diametral gegenüberliegenden Seite mit einer federnd ausgebildeten Zunge versehen ist, welche bei Herstellung der Verbindung zwischen der Abflachung und dem hakenförmigen Ansatz ausfedert, jedoch dann an der Umfangsfläche des Knochennagels anliegt.

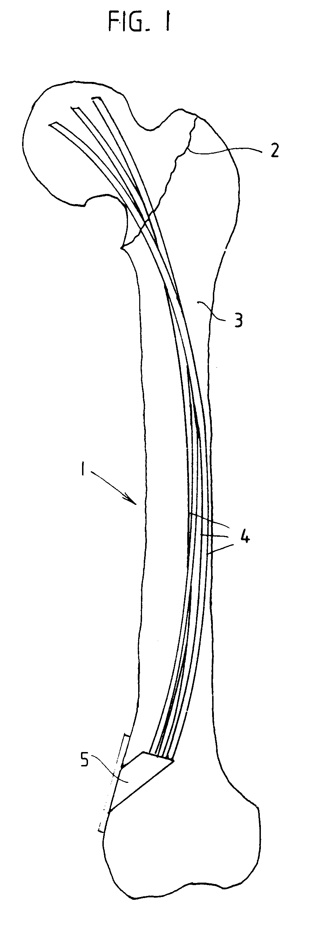

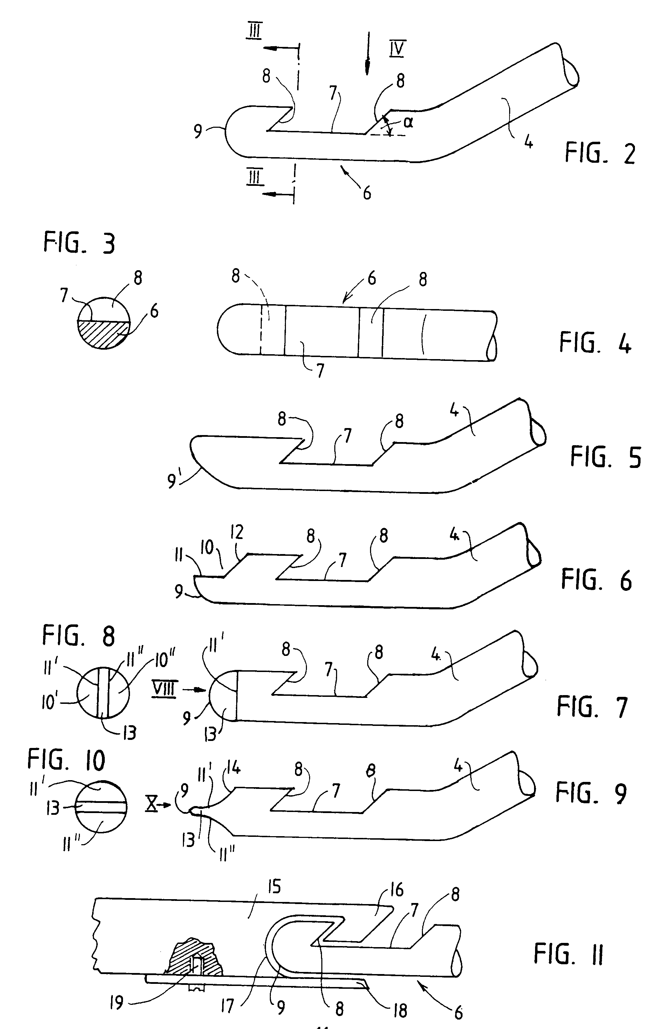

In der Zeichnung ist die Erfindung anhand von Ausführungsbeispielen schematisch veranschaulicht. Fig.1 zeigt einen eine Fraktur aufweisenden Knochen mit drei erfindungsgemäßen Knochennägeln. Fig.2 stellt in größerem Maßstab den distalen Endbereich eines erfindungsgemäßen Knochennagels in Seitenansicht dar. Fig.3 zeigt einen Schnitt nach der Linie III-III in Fig.2 und Fig.4 eine Draufsicht in Richtung des Pfeiles IV in Fig.2. Die Fig.5 bis 7 und 9 stellen abgewandelte Ausführungsformen des distalen Endbereiches eines erfindungsgemäßen Knochennagels in einer Seitenansicht entsprechend der Darstellung in Fig.2 dar. Fig.8 zeigt eine Stirnansicht des distalen Endbereiches des in Fig.7 dargestellten Knochennagels in Richtung des Pfeiles VIII in Fig.7 und Fig.10 eine Stirnansicht des distalen Endbereiches des in Fig.9 dargestellten Knochennagels in Richtung des Pfeiles X in Fig.9. Fig.11 stellt einen Teil des erfindungsgemäßen Werkzeuges im Zusammenwirken mit dem distalen Endbereich eines erfindungsgemäßen Knochennagels dar.

In Fig.1 ist ein Knochen 1 gezeigt, der mit einer Fraktur 2 versehen ist. Zur Reposition und Fixation der Fraktur 2 sind im Markraum 3 des Knochens 1 drei aus elastischem Material bestehende Knochennägel 4 mit rundem Querschnitt eingesetzt, die im proximalen Endbereich gekrümmt sind. Zum Einsetzen dieser Knochennägel wird zunächst der Markraum 3 im Gelenkbereich des Knochens 1 punktförmig eröffnet und anschließend mit einem Fräser oder Bohrer ein Einschlagloch gefräst oder gebohrt, wobei die Lochachse so gewählt ist, daß das Einschlagen der Nägel 4 in der erforderlichen Richtung erfolgen kann. Anschließend wird ein Einsatzstück 5 in das Einschlagloch eingesetzt, worauf die Knochennägel 4 eingeschlagen und verdreht werden, um die Bruchstelle zu reponieren und zu fixieren. Hiezu sind die Knochennägel 4 in ihrem distalen Endbereich 6 mit einem Kupplungsteil versehen, der in den Fig.2 bis 10 näher dargestellt ist.

Wie aus den Fig.2 bis 10 hervorgeht, weist der distale Endbereich 6 denselben Querschnitt auf wie der übrige Knochennagel 4, und besitzt eine Abflachung 7, die über Stirnflächen 8 in den Umfang des Knochennagels übergeht. Die Stirnflächen 8 sind zueinander parallel und verlaufen geneigt zur Abflachung 7, wobei sie mit der Abflachung 7 vorzugsweise einen Winkel von etwa 45° einschließen. Die Neigung der Stirnflächen 8 ist derart, daß diese Stirnflächen 8 vom Umfang des Knochennagels ausgehend zur Abflachung 7 in Richtung zum distalen Nagelende 9 verlaufen.

Durch die erfindungsgemäße Ausbildung der Stirnflächen 8 wird, wie im folgenden in Verbindung mit Fig.11 noch näher erläutert wird, eine sichere Verbindung mit dem mit der Abflachung 7 zusammenwirkenden Teil eines Werkzeuges sichergestellt, ohne daß für die Fixierung dieses Werkzeugteiles zusätzliche Maßnahmen erforderlich sind.

Bei der Ausführungsform nach Fig.2 ist das distale Nagelende 9 kugelsegmentförmig ausgebildet.

Fig.5 zeigt eine Ausführungsform, bei welcher das distale Nagelende im Querschnitt in einer in Nagellängsrichtung verlaufenden, mit der Papierebene zusammenfallenden Normalebene auf die Abflachung 7 von einer zur Abflachung geneigt verlaufende Kurve 9′ begrenzt ist, wobei die Neigung dieser Kurve entgegengesetzt zur Neigung der dem distalen Nagelende benachbarten Stirnfläche 8 verläuft. Diese Ausbildung stellt ein unbehindertes Gleiten des distalen Nagelendes entlang der Wand des Führungskanales eines Einsatzstückes sicher, was bei einer Belastung des Beines, in dessen Markraum sich die Knochennägel befinden, erforderlich ist, um eine Perforation des Gelenkskopfes zu vermeiden.

Fig.6 zeigt eine Ausführungsform, bei welcher das distale Nagelende 9 kugelsegmentförmig ausgebildet ist, jedoch mit einer von der Umfangsfläche des Nagels einspringenden, gegen das distale Nagelende 9 offenen Ausnehmung 10 versehen ist. Die Begrenzungsfläche 11 der Ausnehmung 10 verläuft parallel zur Abflachung 7 und geht über eine geneigte Fläche 12, welche parallel zur Stirnfläche 8 verläuft, in die Umfangsfläche des Knochennagels über. Durch diese Ausbildung wird eine sichere drehfeste Verbindung des Nagels mit einem entsprechend ausgebildeten Werkzeug gewährleistet, wie sie beim Reponieren der Bruchstelle durch Verdrehen des Nagels erforderlich ist.

Die Fig.7 und 8 zeigen eine Ausführungsform, bei welcher an zwei diametral gegenüberliegenden Umfangsflächen von diesen Umfangsflächen einspringende, gegen das Nagelende offene Ausnehmungen 10′,10˝ vorgesehen sind, die einen gegen das distale Nagelende gerichteten Vorsprung 13 begrenzen. Dieser Vorsprung 13 gewährleistet im Zusammenwirken mit einem entsprechend ausgebildeten Werkzeug gleichfalls eine zusätzliche drehfeste Verbindung zwischen diesem Werkzeug und dem Knochennagel. Bei der Ausführungsform nach den Fig.7 und 8 verlaufen die Begrenzungsflächen 11′,11˝ der beiden Ausnehmungen 10′,10˝ senkrecht zur Abflachung 7.

Die Ausführungsform nach den Fig.9 und 10 unterscheidet sich von der Ausführungsform nach den Fig.7 und 8 dadurch, daß hier die Begrenzungsflächen 11′,11˝ parallel zur Abflachung 7 verlaufen. Ferner gehen bei dieser Ausführungsform die Begrenzungsflächen 11′,11˝ über eine geneigte Fläche 14 in die Umfangsfläche des Knochennagels über.

In den Fig.2 bis 10 ist lediglich eine einzige Abflachung 7 mit geneigten Stirnflächen 8 dargestellt. Es genügt auch eine solche einzige Abflachung für die Kupplung des distalen Endbereiches des Knochennagels mit einem entsprechend ausgebildeten Werkzeug. Fallweise ist es jedoch zweckmäßig, wenn dieses Werkzeug an verschiedenen Stellen mit dem distalen Endbereich des Knochennagels gekuppelt werden kann. Zu diesem Zweck können mehrere Abflachungen 7 mit geneigten Stirnflächen 8 im distalen Endbereich vorgesehen sein, und zwar nicht nur in Umfangsrichtung versetzt angeordnet, sondern auch in Nagellängsrichtung versetzt angeordnet.

Fig.11 zeigt den mit dem distalen Endbereich 6 eines Knochennagels zusammenwirkenden Teil eines Werkzeuges 15. Dieses weist einen hakenartigen, der Form der Stirnfläche 8, über welche die Abflachung 7 in die Umfangsfläche des Nagels übergeht, angepaßten Ansatz 16 auf, der diese Stirnfläche 8 hintergreift, und geht über eine der Form des distalen Nagelendes 9 angepaßte gekrümmte Fläche 17 in den Werkzeugschaft über. Dadurch ist der distale Endbereich zwischen der Stirnfläche 8 und dem Nagelende 9 von den genannten Teilen des Werkzeuges umfaßt. Ein solches Werkzeug kann sowohl zum Einschlagen des Knochennagels in den Markkanal als auch zum Zurückschlagen des Knochennagels aus dem Markkanal verwendet werden und benötigt einen geringen Platz, so daß es auch mit dem distalen Endbereich des Knochennagels gekuppelt werden kann, wenn sich dieser innerhalb eines Führungskanales eines Einsatzstückes befindet. Um ein Lösen des Werkzeuges vom distalen Endbereich des Knochennagels mit Sicherheit zu verhindern, ist der Werkzeugschaft an der dem hakenförmigen Ansatz 16 diametral gegenüberliegenden Seite mit einer federnd ausgebildeten Zunge 18, beispielsweise aus Federstahl, versehen, die über eine Schraube 19 mit dem Werkzeugschaft verbunden ist. Diese federnd ausgebildete Zunge federt beim Kupplungsvorgang des Werkzeuges mit dem distalen Endbereich des Knochennagels aus, behindert diesen Kupplungsvorgang somit nicht, legt sich jedoch, sobald der hakenartige Ansatz 16 die Stirnfläche 8 hintergriffen hat, an die Umfangsfläche des Knochennagels an. Da die federnd ausgebildete Zunge 18 verhältnismäßig dünn ausgebildet werden kann, werden durch diese Zunge die Abmessungen des Werkzeuges nur unwesentlich vergrößert.

## Patentansprüche

1. Zumindest in seinem proximalen Endbereich gekrümmter Knochennagel (4) aus elastischem Material mit rundem oder ovalem Querschnitt zur Fixierung von Brüchen im proximalen Oberschenkelbereich, welcher Nagel (4) über ein proximal des Kniegelenkbereiches im Knochen (1) angeordnetes Einschlagloch in den Markraum (3) des Knochens (1) eingeführt werden kann und infolge seiner Elastizität mit dem Krümmungsscheitel an der dem Einschlagloch gegenüberliegenden Wand des Markraumes (3) unter Spannung anliegen kann, und dessen distaler Endbereich denselben Querschnitt aufweist wie der gesamte Nagel (4) und zur Ausbildung als Kupplungsteil für die Herstellung einer allseits drehfesten Verbindung mit einem Werkzeug an zumindest einer Stelle des Nagelumfanges in Abstand von distalen Nagelende (9) mit einer Abflachung (7) versehen ist, dadurch gekennzeichnet, daß zumindest die dem distalen Nagelende (9) unmittelbar benachbarte Stirnfläche (8), über welche die Abflachung (7) in den Umfang des Knochennagels (4) übergeht, vom Umfang des Knochennagels ausgehend zur Abflachung (7) in Richtung zum distalen Nagelende (9) geneigt ausgebildet ist, somit mit dieser Abflachung (7) einen von 90° abweichenden Winkel einschließt.

2. Knochennagel nach Anspruch 1, dadurch gekennzeichnet, daß beide gegenüberliegende Stirnflächen (8), über welche die Abflachung (7) in den Umfang des Knochennagels (4) übergeht, zueinander parallel und zur Abflachung (7) geneigt ausgebildet sind.

3. Knochennagel nach Anspruch 1 oder 2, dadurch gekennzeichnet, daß der Winkel (α), den die geneigte Stirnfläche (8) mit der Abflachung (7) einschließt, zwischen 30° und 60°, vorzugsweise etwa 45°, beträgt.

4. Knochennagel nach einem der Ansprüche 1 bis 3, dadurch gekennzeichnet, daß zwei oder mehrere in Umfangsrichtung des Nagels und/oder in Nagellängsrichtung versetzt angeordnete Abflachungen (7) vorgesehen sind.

5. Knochennagel nach einem der Ansprüche 1 bis 4, dadurch gekennzeichnet, daß das distale Nagelende (9) mit wenigstens einer von der Umfangsfläche des Nagels einspringenden, gegen das Nagelende offenen Ausnehmung (10,10′,10˝) versehen ist.

6. Knochennagel nach Anspruch 5, dadurch gekennzeichnet, daß an zwei diametral gegenüberliegenden Umfangsflächen des Nagels Ausnehmungen (10′,10˝) vorgesehen sind, von welchen ein gegen das distale Nagelende (9) gerichteter Vorsprung (13) begrenzt ist.

7. Knochennagel nach Anspruch 5 oder 6, dadurch gekennzeichnet, daß die Begrenzungsfläche (11,11′,11˝,14) der Ausnehmung bzw. Ausnehmungen (10,10′,10˝) zumindest teilweise etwa parallel zur Abflachung (7) verläuft (Fig.6, Fig.9 und Fig.10).

8. Knochennagel nach Anspruch 5 oder 6, dadurch gekennzeichnet, daß die Begrenzungsfläche (11′,11˝) der Ausnehmung bzw. Ausnehmungen (10′,10˝) zumindest teilweise senkrecht zur Abflachung (7) verläuft (Fig.9 und 10).

9. Knochennagel nach einem der Ansprüche 5 bis 8, dadurch gekennzeichnet, daß die Begrenzungsfläche (11′,11˝) der Ausnehmung(en) (10′,10˝) über eine Abrundung (14) in die Umfangsfläche des Nagels übergeht (Fig.9 und 10).

10. Knochennagel nach einem der Ansprüche 5 bis 8, dadurch gekennzeichnet, daß die Begrenzungsfläche (11) der Ausnehmung (10) über eine geneigte Fläche (12) in die Umfangsfläche des Nagels übergeht, deren Neigung vorzugsweise gleich ist jener der geneigten Stirnfläche (8), über welche die Abflachung (7) in die Umfangsfläche des Nagels übergeht.

11. Knochennagel nach einem der Ansprüche 1 bis 10, dadurch gekennzeichnet, daß das distale Nagelende im Querschnitt in einer in Nagellängsrichtung verlaufenden Normalebene auf die Abflachung (7) von einer geneigt zur Abflachung (7) verlaufenden Kurve (9′) begrenzt ist.

12. Knochennagel nach Anspruch 11, dadurch gekennzeichnet, daß die Neigung der Kurve (9′) entgegengesetzt zur Neigung der dem distalen Nagelende benachbarten Stirnfläche (8) verläuft.

13. Kombination eines Werkzeuges und eines Knochennagels nach einem der Ansprüche 1 bis 12, dadurch gekennzeichnet, daß der Werkzeugschaft (15) einen hakenartigen, der Form wenigstens einer Stirnfläche (8), über welche die Abflachung (7) in die Umfangsfläche des Nagels übergeht, angepaßten Ansatz (16) aufweist.

14. Kombination nach Anspruch 13, dadurch gekennzeichnet, daß der hakenartige Ansatz (16) über eine der Form des distalen Nagelendes (9) angepaßte gekrümmte Fläche (17) in den Werkzeugschaft (15) übergeht.

15. Kombination nach Anspruch 13 oder 14, dadurch gekennzeichnet, daß der Werkzeugschaft (15) an der dem hakenartigen Ansatz (16) diametral gegenüberliegenden Seite mit einer federnd ausgebildeten Zunge (18) versehen ist.

## Claims

1. A bone nail (4) of elastic material, having round or oval cross section and being bent at least in its proximal end portion for fixing fractures in the proximal femural area, which nail (4) is insertable via an impact hole proximal to the knee joint area of the bone (1) into the medullary canal (3) of the bone (1) and due to its elasticity can abut under tension the wall of the medullary canal (3) opposite the impact hole with the apex of curvature, and the distal end portion of which has the same cross section as the entire nail (4) and is provided on at least on site of the nail circumference spaced apart from the distal nail end (9) with a flattening (7) for forming a coupling element for all-round non-rotatable connection with a tool, characterized in that at least that front face (8) which immediately neighbours the distal nail end (9) and by which the flattening (7) merges with the circumference of the bone nail (4), is inclined starting from the circumference of the bone nail to the flattening (7) in direction towards the distal nail end (9), and therefore includes with this flattening (7) an angle deviating from 90°.

2. Bone nail according to claim 1, characterized in that both opposing front faces (8) via which the flattening (7) merges with the circumference of the bone nail (4), extend parallel to each other and inclined towards the flattening (7).

3. Bone nail according to claim 1 or 2, characterized in that the angle (α) included between the inclined front face (4) and the flattening (7) amounts to between 30° and 60°, preferably about 45°.

4. Bone nail according to any of claims 1 to 3, characterized in that two or more flattenings (7) are provided that are offset in circumferential direction of the nail and/or in longitudinal direction of the nail.

5. Bone nail according to any of claims 1 to 4, characterized in that the distal nail end (9) is provided with at least one recess (10, 10′,10˝) extending back from the circumferential surface of the nail and being open towards the nail end.

6. Bone nail according to claim 5, characterized in that recesses (10′,10˝) are provided on two peripheral surfaces of the nail disposed diametrically opposing each other, a projection (13) directed towards the distal nail end (9) being defined by said recesses.

7. Bone nail according to claim 5 or 6, characterized in that the boundary surface (11,11′,11˝,14) of the recess or the recesses (10, 10′,10˝) extends at least partially about parallel to the flattening (7) (Fig.6, Fig.9 and Fig.10).

8. Bone nail according to claim 5 or 6, characterized in that the boundary surface (11′,11˝) of the recess or recesses (10′,10˝) extends at least partially perpendicularly to the flattening (7) (Fig.9 and 10).

9. Bone nail according to any of claims 5 to 8, characterized in that the boundary surface (11′,11˝) of the recess(es) (10′,10˝) merges via a rounding (14) with the circumferential surface of the nail (Fig.9 and 10).

10. Bone nail according to any of claims 5 to 8, characterized in that the boundary surface (11) of the recess (10) merges via an inclined surface with the circumferential surface of the nail, the inclination thereof being preferably equal to that of the inclined front face (8) via which the flattening (7) merges with the circumferential surface of the nail.

11. Bone nail according to any of claims 1 to 10, characterized in that the distal nail end is in cross section confined by a curve (9′) extending inclined to the flattening (7) in a plane perpendicularly to the flattening (7) and extending in longitudinal direction of the nail.

12. Bone nail according to claim 11, characterized in that the inclination of the curve (9′) extends opposite to the inclination of the front face (8) neighbouring the distal nail end.

13. A combination of a tool and a bone nail according to any of claims 1 to 12, characterized in that the tool shaft (15) comprises a hook-like protrusion (16) that corresponds to the shape of at least one front face (8) via which the flattening (7) merges with the circumferential surface of the nail.

14. Combination according to claim 13, characterized in that the hook-like protrusion (16) merges with the tool shaft (15) via a curved surface (17) adapted to the shape of the distal nail end (9).

15. Combination according to claim 13 or 14, characterized in that the tool shaft (15) is provided with a resilient tongue (18) on the side diametrically opposing the hook-shaped protrusion (16).

## Revendications

1. Broche pour os (4), courbée au moins vers son extrémité proximale, en matériau élastique avec section circulaire ou ovale pour la fixation de fractures dans la partie proximale de la cuisse, laquelle broche (4) peut être introduite dans le canal médullaire (3) de l'os (1) par un trou d'insertion pratiqué à proximité de la zone d'articulation du genou, et s'appliquer par le sommet de courbure contre la paroi du canal médullaire opposée au trou d'insertion, en raison de son élasticité, et dont la zone d'extrémité distale présente la même section que la totalité de la broche (4), et pourvue en au moins un emplacement du pourtour de broche, à distance de l'extrémité distale (9) de broche, d'un méplat (7) pour former une partie d'accouplement pour la réalisation d'une liaison partout fixe en rotation avec un outil, caractérisée en ce que au moins la surface frontale (8) située à proximité immédiate de l'extrémité distale de broche (9) par laquelle s'effectue la transition du méplat (7) vers le pourtour de la broche pour os (4), est conformée de façon inclinée en partant du pourtour de la broche pour os vers le méplat (7) en direction de l'extrémité distale de la broche pour os (9), de sorte qu'elle forme, avec ledit méplat (7), un angle différent de 90°.

2. Broche pour os selon la revendication 1, caractérisée en ce que les deux surfaces frontales opposées (8), par lesquelles s'effectue la transition du méplat (7) vers le pourtour de la broche pour os (4), sont parallèles entre elles et inclinées par rapport au méplat (7).

3. Broche pour os selon la revendication 1 ou 2, caractérisée en ce que l'angle (α), qui incorpore la surface frontale inclinée (8) et le méplat (7) est compris entre 30° et 60°, de préférence environ 45°.

4. Broche pour os selon l'une des revendications 1 à 3, caractérisée en ce qu'il est prévu deux méplats (7), ou davantage, disposés de façon décalée en direction de pourtour de la broche et/ou en direction longitudinale de broche.

5. Broche pour os selon l'une des revendications 1 à 4, caractérisée en ce que l'extrémité distale de broche (9) présente au moins un évidement (10, 10′, 10˝) s'étendant depuis la surface périphérique de la broche et s'ouvrant vers l'extrémité de broche.

6. Broche pour os selon la revendication 5, caractérisée en ce que des évidements (10′, 10˝) sont prévus en deux surfaces périphériques diamétralement opposées de la broche, et qui délimitent un prolongement (13) orienté vers l'extrémité distale de broche (9).

7. Broche pour os selon la revendication 5 ou 6, caractérisée en ce que les surfaces (11, 11′, 11˝, 14) délimitant le ou les évidements (10, 10′, 10˝) s'étendent, au moins en partie, environ parallèlement au méplat (7) (Fig. 6, Fig. 9 et Fig. 10).

8. Broche pour os selon la revendication 5 ou 6, caractérisée en ce que les surfaces (11′, 11˝) délimitant le ou les évidements (10′, 10˝) s'étendent au moins en partie perpendiculairement au méplat (7) (Fig. 9 et 10).

9. Broche pour os selon l'une des revendications 5 à 8, caractérisée en ce que les surfaces (11′, 11˝) délimitant le ou les évidements (10′, 10˝) se raccordent à la surface périphérique de broche par un arrondi (14) (Fig. 9 et 10).

10. Broche pour os selon l'une des revendications 5 à 8, caractérisée en ce que les surfaces (11) délimitant l'évidement (10) se raccordent à la surface périphérique de la broche par une surface inclinée (12), dont l'inclinaison est de préférence égale à celle de la surface frontale inclinée (8) par laquelle le méplat (7) rejoint la surface périphérique de broche.

11. Broche pour os selon l'une quelconque des revendications 1 à 10, caractérisée en ce que l'extrémité distale de broche est, en section dans un plan normal au méplat- (7) et s'étendant en direction longitudinale de broche, limitée par une courbe (9′) s'étendant de façon inclinée par rapport au méplat (7).

12. Broche pour os selon la revendication 11, caractérisée en ce que l'inclinaison de la courbe (9') s'étend de façon contraire à l'inclinaison de la surface frontale (8) proche de l'extrémité distale de broche.

13. Combinaison d'un outil et d'une broche pour os selon l'une des revendications 1 à 12, caractérisée en ce que la tige d'outil (15) présente un prolongement (16) en forme de crochet, adapté à la forme d'au moins une surface frontale (8) par laquelle s'effectue la transition du méplat (7) vers la surface périphérique de broche.

14. Combinaison selon la revendication 13, caractérisée en ce que le prolongement (16) en forme de crochet se raccorde dans la tige d'outil (15) par une surface courbée (17) adaptée à la forme de l'extrémité distale de broche (9).

15. Combinaison selon la revendication 13 ou 14, caractérisée en ce que la tige d'outil (15) est pourvue, sur son côté diamétralement opposé au prolongement en forme de crochet (16), d'une languette (18) réalisée pour être élastique.
